(19) Europäisches Patentamt
European Patent Office
Office européen des brevets

(11) Numéro de publication: **0 190 523 B1**

# (12) FASCICULE DE BREVET EUROPEEN

(45) Date de publication du fascicule du brevet: **20.04.88**

(21) Numéro de dépôt: **85402486.6**

(22) Date de dépôt: **13.12.85**

(51) Int. Cl.⁴: **C 07 B 39/00**, C 07 C 43/225, C 07 C 25/13, C 07 C 21/24, C 07 C 19/08, C 07 C 17/18, C 07 C 41/22

(54) **Procédé de préparation de composés porteurs d'un groupe difluorométhylène ou trifluorométhyle.**

(30) Priorité: **26.12.84 FR 8419799**

(43) Date de publication de la demande: **13.08.86 Bulletin 86/33**

(45) Mention de la délivrance du brevet: **20.04.88 Bulletin 88/16**

(84) Etats contractants désignés: **AT BE CH DE FR GB IT LI LU NL SE**

(56) Documents cités:
**CH - A - 401 940**
**US - A - 4 093 665**

(73) Titulaire: **RHONE-POULENC CHIMIE, 25, quai Paul Doumer, F-92408 Courbevoie Cédex (FR)**

(72) Inventeur: **Desbois, Michel, 526, Chemin du Bois, F-69140 Rillieux (FR)**

(74) Mandataire: **Cazes, Jean-Marie et al, RHONE-POULENC INTERSERVICES Service Brevets Chimie 25, quai Paul Doumer, F-92408 Courbevoie Cédex (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 190 523 B1

## Description

La présente invention concerne un procédé de préparation de composés porteurs d'un groupe difluorométhylène ou trifluorométhyle. Elle concerne plus particulièrement un procédé de préparation de composés porteurs d'un groupe difluorométhylène ou trifluorométhyle obtenus par fluoration de composés porteurs d'un groupe carbonyle.

Sheppard a décrit depuis longtemps déjà (Journal ou Organic Chemistry 29, 1, 1-11 1964) la fluoration des halogénures d'acides à l'aide de tétrafluorure de soufre et d'acide fluorhydrique. D'une part, la méthode de préparation utilisant le tétrafluorure de soufre, présente de grosses contraintes du point de vue sécurité, à cause de la très forte toxicité de $SF_4$. D'autre part, $SF_4$ n'est pas un produit disponible industriellement. Ainsi, toute exploitation industrielle par cette voie est à éviter.

La méthode décrite par Sheppard a été perfectionnée par l'addition de cocatalyseurs divers tels que $BF_3$, $TF_5$, $TiF_4$ (brevet US N° 4 288 601) mais ces cocatalyseurs ne changent adsolument pas les problèmes de toxicité et de disponibilité.

Il est encore connu d'après le Journal of Organic Chemistry 20 (1982) 581-587 de procéder à la fluoration des groupes carbonyles ou carboxyles aliphatiques au moyen de catalyseurs à base d'hexafluorure de tungstène ou d'hexafluorure de molybdène parfois associé à du trifluorure de bore.

Ces catalyseurs ne sont pas utilisables industriellement car, d'une part, ils ne sont pas disponibles sur le marché, d'autre part, ils ne réalisent l'échange du groupe carbonyle en groupe difluorométhylène que sur les cétones ou les aldéhydes.

Ils ne répondent donc pas aux objectifs de la présente invention.

La présente invention a su vaincre les inconvénients de l'art antérieur et a pour objet un procédé de préparation de composés comportant un groupe difluorométhylène ou trifluorométhyle, caractérisé en ce qu'on met en présence dans l'acide fluorhydrique liquide anhydre, soit un composé porteur d'un groupe carbonyle choisi parmi les acides, les halogénures d'acides, les amides, les cétones et soit tous les dérivés porteurs d'un groupe trihalogénoalkylcarbonyle avec du trifluorure de bore en quantité telle que la pression absolue de trifluorure de bore dans l'enceinte réactionnelle soit d'au moins un bar.

On entend par composés porteurs d'un groupe carbonyle, les composés de formule générale R CO X dans laquelle, lorsque R représente un groupe choisi parmi les radicaux alkyle linéaire ou ramifié, halogénoalkyle, phényle éventuellement substitué par un radical alkyle, halogéno, alcoxy, halogénoalkyle, phényle, phénoxy, nitro, amino, X est un groupe choisi parmi les radicaux hydroxy, halogéno, amino, alkyle, halogénoalkyle, phényle éventuellement substitué et lorsque R est un radical perhalogénoalkyle, X représente un groupe choisi parmi les radicaux hydroxy, halogéno, alkyle, halogénoalkyle, alcoxy, halogénoalcoxy, phényle, halogénophényle, phénoxy, halogénophénoxy, amino, alkylthio, phénylthio, halogénoalkylthio, halogénophénylthio.

Les composés porteurs d'un groupe carbonyle préférés, utilisés dans le procédé de l'invention sont les halogénures d'acides et les esters d'acides aliphatiques perfluorés et tout particulièrement les esters de l'acide trifluoracétique.

Le trifluorure de bore présente de grands avantages par rapport aux catalyseurs utilisés dans l'art antérieur. C'est un produit industriel, donc disponible sur le marché, ce qui le différencie de l'hexafluorure de tungstène et du tétrafluorure de soufre. Il présente, d'autre part, l'avantage d'être gazeux ce qui permet de la séparer facilement et de le recycler en fin de réaction.

Il est préférable de travailler avec une quantité de $BF_3$ telle que la pression absolue de $BF_3$ dans l'enceinte réactionnelle soit comprise entre 5 et 50 bars.

Avantageusement, le rapport molaire de l'acide fluorhydrique au dérivé porteur d'un groupe carbonyle est compris entre 5 et 50. Encore plus préférentiellement, il est compris entre 10 et 30.

La température de la réaction est comprise de préférence entre 0 et 150°C et encore plus préférentiellement entre 20 et 80°C.

La réaction peut avoir lieu en présence de solvants. Parmi les solvants utilisables, on peut citer mais non limitativement, $CCl_4$, $CHCl_3$, $CFCl_2$-$CF_2Cl$.

Les dérivés fluorés obtenus selon le procédé de l'invention répondent à la formule générale $R'CF_2X$ dans laquelle R' représente un groupe alkyle linéaire ou ramifié, aryle, halogénoalkyle, halogénophényle, alkylphényle, halogénoalkylphényle, et dans laquelle X est échangé avec les atomes de fluor du milieu réactionnel lorsqu'il représentait un groupe hydroxy ou halogène.

Parmi les produits obtenus selon le procédé de l'invention, on peut citer les produits suivants:

trifluorométhyl-2 propane, chloro-4 trifluorométhylbenzène, trifluorométhylbenzène, nitro-4 trifluorométhylbenzène, dichloro-3, 4 trifluorométhylbenzène, nitro-2 trifluorométhylbenzène, difluoro-2,6 trifluorométhylbenzène, chloro-2 fluoro-6 trifluorométhylbenzène, chloro-4 pentafluoroéthoxybenzène, pentafluoroéthoxybenzène, difluoro-α,α trichloro-β,β,β éthoxybenzène, phénoxy-4 trifluorométhylbenzène, méthyl-3 trifluorométhylbenzène, méthyl-2 trifluorométhylbenzène, phényl-4 trifluorométhylbenzène, pentafluoroéthyl trifluoro-β,β,β éthyl éther, difluoro-α,α éthylbenzène, diphényldifluorométhane, bis (fluoro-4 phényl) difluoro méthane, fluoro-4 pentafluoroéthoxybenzène, trifluorométhyl-3 pentafluoroéthoxybenzène, trifluorométhyl-4 pentafluoroéthoxybenzène, chloro-2 trifluorométhyl-4 pentafluoroéthoxybenzène, trifluorométhoxy-4 pentafluoroéthoxybenzène.

Les dérivés fluorés obtenus selon le procédé de l'invention sont utilisés comme intermédiaires de synthèse dans l'industrie pharmaceutique, phytosanitaire, des colorants, comme anesthésiques, (Kirk Othmer, 2, p. 684-689) ou comme fluides thermiques et lubrifiants.

L'invention va être maintenant plus complètement décrite sans aucun caractère limitatif du domaine de l'invention à l'aide des exemples qui vont suivre.

*Exemple 1*

Dans un réacteur inox de 250 ml agité par barreau aimanté et refroidi par un bain d'eau glacée, on introduit 100 g de HF anhydre et 31,7 g (0,2 m) de chlorure de p-fluorobenzoyle. Après dégazage du HCl formé, le réacteur est fermé, porté à la pression de 20 bars (à 15°C) par du $BF_3$ gazeux et chauffé 17 heures à 50°C. Après refroidissement et décompression, le mélange brut réactionnel obtenu est coulé sur 200 g de glace pilée, puis extrait par 100 $cm^3$ de $CH_2Cl_2$. La phase organique recueillie est lavée par 2 fois 100 $cm^3$ d'eau et séchée.

Après analyses chromatographie phase gazeuse et spectrométrie de masse, on note la composition suivante:

CF3
COF
COOH
F
F
F

≃ 21,5% ≃ 74% ≃ 3,2 %

*Exemple 2*

Dans un réacteur inox de 250 ml muni d'un agitateur magnétique, on introduit, à 0°C, successivement 100 g (5 m) de HF anhydre et 58 g (0,55 m) de chlorure d'isobutyroyle. On laisse l'acide chlorhydrique produit par la réaction de fluoration du chlorure d'isobutyroyle en fluorure d'isobutyroyle se dégager (25 mn). Le réacteur est alors fermé puis porté à la pression de 10 bars par du $BF_3$ gazeux. On laisse réagir 24 heures à 50°C. Après réaction, le réacteur est refroidi à 0°C, décompressé et le mélange réactionnel coule sur 150 g de glace pilée. La solution ainsi obtenue est immédiatement extraite par 2 fois 100 ml de $CH_2Cl_2$.

Les analyses effectuées par couplage chromatographie phase gazeuse et spectrométrie de masse sur cette solution organique mettent en évidence la présence de trifluorométhyl-2 propane (M = 112).

*Exemple 3*

Dans un réacteur inox de 250 ml agité par barreau aimanté et refroidi par un bain d'eau glacée, on introduit sous atmosphère inerte 100 g de HF anhydre et 67,2 g (0,3 m) de trifluoroacétate de p-chlorophényle.

Le réacteur est fermé, porté à la pression de 15 bars (à 30°C) par du $BF_3$ gazeux, puis chauffé à 80°C pendant 3 heures (pression 25 bars).

Après refroidissement et décompression, le mélange brut réactionnel obtenu est coulé sur 200 g de glace pilée, puis extrait par 100 $cm^3$ de $CH_2Cl_2$.

La phase organique recueillie est lavée par 2 fois 100 $cm^3$ d'eau et séchée.

Après analyse chromatographie phase gazeuse, on note la composition suivante:

OCOCF3
OCF2CF2
OH
Cl
Cl
Cl

≃ 53,5% ≃ 18% ≃ 27 %

*Exemple comparatif N° 3 A*

Selon le mode opératoire de l'exemple 3, on met en présence:

OCOCF3

BF3

0,1 mole = 19 g

19 bars à 14°C

On chauffe à 80°C pendant 2 h 30 à une pression maximale de 24 bars.

On n'obtient aucune trace de pentafluoroéthoxybenzène.

*Exemple comparatif N° 3 B*

Selon le même mode opératoire qu'à l'exemple 3, on met en présence:

OCOCF3

0,1 mole = 19 g

HF anhydre 5 moles = 100 g

On chauffe à 80°C pendant 23 h 30 à une pression maximale de 5 bars.

On n'obtient aucune trace de pentafluoroéthoxybenzène.

*Exemple 4*

Le mode opératoire utilisé est identique à celui décrit dans l'exemple 1 avec les produits et les conditions suivants:

COOH
F

0,1 mole = 14 g

HF anhydre 5 moles = 100 g

$BF_3$ 20 bars à 20°C

On chauffe à 50°C pendant 20 heures à une pression maximale de 28 bars.

Après traitement, les analyses par chromatographie phase gazeuse et spectrométrie de masse (couplage) mettent nettement en évidence la présence de

F — CF$_3$.

*Exemple 5*

Le mode opératoire utilisé est identique à celui décrit dans l'exemple 3 avec les conditions suivantes:

OCOCF3
Cl

0,2 mole = 44,9 g

| | |
|---|---|
| HF | 5 moles = 100 g |
| $BF_3$ | 40 bars à 20°C |
| T° | 20°C |
| Durée | 18 heures |

Après analyse chromatographie phase gazeuse, on note la composition suivante:

OCOCF3
OCF2CF3
OH
Cl
Cl
Cl

32,3% 3,7% 61,7%

*Exemple 6*

Le mode opératoire utilisé est identique à celui décrit dans l'exemple 1 avec les produits et les conditions suivants:

$COCH_3$

| | |
|---|---|
| | 0,1 mole = 12 g |
| HF anhydre | 5 moles = 100 g |
| $BF_3$ | 20 bars à 20°C |
| T° | 50°C |
| Durée | 3 H 45 |

Après traitement, les analyses par résonnance magnétique nucléaire du fluor 19 montrent la présence d'α α-difluoro éthyl benzène.

*Exemple 7*

Le mode opératoire utilisé est identique à celui décrit dans l'exemple 1 avec les produits et les conditions suivants:

CO

| | |
|---|---|
| | 0,1 mole = 18,2 g |
| HF anhydre | 5 moles = 100 g |
| $CCl_4$ | 0,2 mole = 30,8 g |
| $BF_3$ | 6 bars à 20°C |
| T° | 80°C |
| Durée | 24 heures |

Après traitement, les analyses par couplage chromatographie phase gazeuse/masse et résonnance magnétique nucléaire du fluor 19 nous montrent la présence de diphényldifluorométhane (≃ 5 %).

*Exemple 8*

Le mode opératoire utilisé est identique à celui décrit dans l'exemple 1 avec les produits et les conditions suivants:

OCOCF3
F

0,2 mole = 41,6 g

| | |
|---|---|
| HF | 5 moles = 100 g |
| $BF_3$ | 30 bars à 20°C |
| T° | 40°C |
| Durée | 22 heures |

Après traitements, les analyses par couplage chromatographie phase gazeuse/masse nous montrent la présence de fluoro-4 pentafluoroéthoxybenzène.

*Exemple 9*

Le mode opératoire utilisé est identique à celui décrit dans l'exemple 1 avec les produits et les conditions suivants:

$CF_3$ / $OCOCF_3$

0,15 mole = 38,7 g

| | |
|---|---|
| HF | 4 moles = 80 g |
| $BF_3$ | 25 bars à 20°C |
| T° | 50°C |
| Durée | 15 heures |

Après traitement, les analyses par couplage chromatographie phase gazeuse/masse nous montrent la présence de trifluorométhyl-3 pentafluoroéthoxybenzène.

**Revendications**

1. Procédé de préparation de composés comportant un groupe difluorométhylène ou trifluorométhyle, caractérisé en ce qu'on met en présence, dans l'acide fluorhydrique liquide anhydre, un composé

porteur d'un groupe carbonyle choisi parmi soit les acides, les halogénures d'acides, les amides, les cétones et soit tous les dérivés comportant le radical perhalogénalhylcarbonyle avec du trifluorure de bore en quantité telle que la pression absolue de trifluorure de bore dans l'enceinte réactionnelle soit d'au moins un bar.

2. Procédé selon la revendication 1 caractérisé en ce que le composé porteur d'un groupe carbonyle a pour formule générale R CO X dans laquelle, lorsque R représente un groupe choisi parmi les radicaux alkyle linéaire ou ramifié, halogénoalkyle, phényle éventuellement substitué par un radical alkyle, halogéno, alcoxy, halogénoalkyle, phényle, phénoxy, nitro, amino, X est un groupe choisi parmi les radicaux hydroxy, halogéno, amino, alkyle, halogénoalkyle, phényle éventuellement substitué et lorsque R est un radical perhalogénoalkyle, X représente un groupe choisi parmi les radicaux hydroxy, halogéno, alkyle, halogénoalkyle, alcoxy, halogénoalcoxy, phényle, halogénophényle, phénoxy, halogénophénoxy, amino, alkylthio, halogénoalkylthio, phénylthio, halogénophénylthio.

3. Procédé selon la revendication 2 caractérisé en ce que le composé porteur d'un groupe carbonyle est un halogénure d'acide ou un ester d'acide perfluoré.

4. Procédé selon la revendication 3 caractérisé en ce que l'ester d'acide perfluoré est un ester de l'acide trifluoracétique.

5. Procédé selon la revendication 1 caractérisé en ce que le rapport molaire de l'acide fluorhydrique au dérivé porteur d'un groupe carbonyle est compris entre 5 et 50 et de préférence entre 10 et 30.

6. Procédé selon la revendication 1 caractérisé en ce que la pression absolue de trifluorure de bore est comprise entre 5 et 50 bars.

7. Procédé selon la revendication 1 caractérisé en ce que la température de réaction est comprise entre 0 et 150°C et de préférence entre 20 et 80°C.

8. Procédé selon la revendication 1 caractérisé en ce qu'on ajoute un solvant.

9. Procédé selon la revendication 8 caractérisé en ce que le solvant est choisi parmi $CCl_4$, $CHCl_3$, $CFCl_2$-$CF_2Cl$.

**Patentansprüche**

1. Verfahren zur Herstellung von Verbindungen, die eine Difluormethylen- oder Trifluormethylgruppe aufweisen, dadurch gekennzeichnet, dass man in flüssiger wasserfreier Fluorwasserstoffsäure eine Verbindung, die eine Carbonylgruppe trägt ausgewählt aus entweder den Säuren, den Säurehalogeniden, den Amiden, den Ketonen oder all denjenigen Verbindungen, die einen Perhalogenalkylcarbonylrest aufweisen, mit einer solchen Menge Bortrifluorid umsetzt, dass der absolute Druck an Bortrifluorid im Reaktionsbehälter wenigstens 1 bar beträgt.

2. Verfahren nach Anspruch 1, dadurch gekennzeichnet, dass die Verbindung, die eine Carbonylgruppe trägt der allgemeinen Formel R CO X entspricht, in der, wenn R eine Gruppe ausgewählt aus den linearen oder verzweigten Alkylresten, Halogenalkylresten, Phenylresten gegebenenfalls substituiert mit einem Alkyl-, Halogen-, Alkoxy-, Halogenalkyl-, Phenyl-, Phenoxy-, Nitro-, Aminorest, ist, X eine Gruppe ausgewählt aus den Resten Hydroxy, Halogen, Amino, Alkyl, Halogenalkyl, Phenyl gegebenenfalls substituiert, ist, und wenn R ein Perhalogenalkylrest ist, X eine Gruppe ausgewählt aus den Resten Hydroxy, Halogen, Alkyl, Halogenalkyl, Alkoxy, Halogenalkoxy, Phenyl, Halogenphenyl, Phenoxy, Halogenphenoxy, Amino, Alkylthio, Halogenalkylthio, Phenylthio, Halogenphenylthio ist.

3. Verfahren nach Anspruch 2, dadurch gekennzeichnet, dass die Verbindung, die eine Carbonylgruppe trägt ein Säurehalogenid oder ein Ester einer perfluorierten Säure ist.

4. Verfahren nach Anspruch 3, dadurch gekennzeichnet, dass der Ester der perfluorierten Säure ein Ester der Trifluoressigsäure ist.

5. Verfahren nach Anspruch 1, dadurch gekennzeichnet, dass das molare Verhältnis von Fluorwasserstoffsäure zu der Verbindung, die eine Carbonylgruppe trägt, zwischen 5 und 50 und vorzugsweise zwischen 10 und 30 liegt.

6. Verfahren nach Anspruch 1, dadurch gekennzeichnet, dass der absolute Druck an Bortrifluorid zwischen 5 und 50 bar liegt.

7. Verfahren nach Anspruch 1, dadurch gekennzeichnet, dass die Reaktionstemperatur zwischen 0 und 150°C und vorzugsweise zwischen 20 und 80°C beträgt.

8. Verfahren nach Anspruch 1, dadurch gekennzeichnet, dass man ein Solvens zugibt.

9. Verfahren nach Anspruch 8, dadurch gekennzeichnet, dass das Solvens ausgewählt wird aus $CCl_4$, $CHCl_3$, $CFCl_2$-$CF_2Cl$.

**Claims**

1. Process for the preparation of compounds containing a difluoromethylene or trifluoromethyl group, characterized in that a compound carrying a carbonyl group chosen from amongst either acids, acid halides, amides and ketones or all derivatives containing a perhaloalkylcarbonyl radical is brought into contact, in anhydrous liquid hydrofluoric acid, with boron trifluoride in a quantity such that the absolute pressure of boron trifluoride in the reaction chamber is at least one bar.

2. Process according to Claim 1, characterized in that the compound carrying a carbonyl group has the general formula RCOX in which, when R represents a group chosen from amongst a straight-chain or branched alkyl radical, a haloalkyl radical and a phenyl radical optionally substituted with an alkyl, halo, alkoxy, haloalkyl, phenyl, phenoxy, nitro or amino radical, X is a group chosen from amongst hydroxy, halo, amino alkyl, haloalkyl and optionally substituted phenyl radicals and when R is a perhaloalkyl radical, X represents a group chosen from amongst hydroxy, halo, alkyl, haloalkyl, alkoxy, haloalkoxy, phenyl, halophenyl, phenoxy, halophenoxy, amino, alkylthio, haloalkylthio, phenylthio and halophenylthio radicals.

3. Process according to Claim 2, characterized in

that the compound carrying a carbonyl group is an acid halide or a perfluorinated acid ester.

4. Process according to Claim 3, characterized in that the perfluorinated acid ester is a trifluoroacetic acid ester.

5. Process according to Claim 1, characterized in that the molar ratio of hydrofluoric acid to the derivative carrying a carbonyl group is between 5 and 50 and preferably between 10 and 30.

6. Process according to Claim 1, characterized in that the absolute pressure of boron trifluoride is between 5 and 50 bars.

7. Process according to Claim 1, characterized in that the reaction temperature is between 0 and 150°C and preferably between 20 and 80°C.

8. Process according to Claim 1, characterized in that a solvent is added.

9. Process according to Claim 8, characterized in that the solvent is chosen from amongst $CCl_4$, $CHCl_3$, and $CFCl_2$-$CF_2Cl$.